# EUROPEAN PATENT SPECIFICATION

(11) **EP 1 877 106 B1**
(45) Date of publication and mention of the grant of the patent: **24.06.2009**
(21) Application number: 06721930.3
(22) Date of filing: 25.04.2006
(51) Int. Cl.: A61L 9/12

(54) **VOLATILE LIQUID DISSEMINATION APPARATUS**
VORRICHTUNG ZUR VERTEILUNG VON FLÜCHTIGEN FLÜSSIGKEITEN
DIFFUSEUR DE LIQUIDES VOLATILES

(30) Priority: 28.04.2005 GB 0508544
(43) Date of publication of application: 16.01.2008
(73) Proprietor: Givaudan SA, 1214 Vernier (CH)
(72) Inventor: BROWN, Colin, Bracknell Berkshire RG42 2BD (GB); NAISH, Guy Edward, Bicester Oxfordshire OX26 3WE (GB)
(74) Representative: Givaudan Patents
(86) International application number: PCT/CH2006/000228
(87) International publication number: WO 2006/114013

(56) References cited:
- WO-A-98/32671
- WO-A-20/04096299
- DE-A1- 2 631 309
- DE-U1- 29 609 511
- US-A1- 2002 066 750

## Description

This invention relates to an apparatus for disseminating a volatile liquid into an atmosphere.

Many commercially-available apparatus for the dissemination of volatile liquids, such as fragrances and insecticides, into an atmosphere comprise a reservoir of volatile liquid and, extending therefrom, a liquid transfer member, typically a porous wick. In a more recent development, it has been proposed to replace the wick with an external capillary member, that is, a member bearing external channels of capillary dimensions. Such members have the advantage of avoiding the undesirable fractionating effect that occurs particularly with multi-component fragrances, with one component evaporating into the atmosphere before the others. However, apparatus utilising this technology are prone to leakage during transport, and this has been a major factor in the lack of acceptance of this otherwise superior transfer member technology.

Solutions to this problem have been proposed. For example, in one case, the liquid reservoir is sealed by a frangible seal or puncturable foil or membrane, which is punctured when the apparatus is put into use. The problem with this use is that the reservoir cannot again be sealed and the apparatus must be used until exhausted.

It has now been found that this problem can be overcome by a particular design of apparatus, which apparatus can be used with any liquid transfer member. The invention therefore provides an apparatus according to claim 1.

The invention additionally provides a method according to claim 10.

The reservoir may be any suitable reservoir, made of any suitable material for the long-term retention of the desired volatile liquid, for example, of plastics, glass, metal or ceramics. Plastics are especially useful because of their easy mouldability in a variety of decorative or practical shapes and colours.

The liquid transfer member may be any member capable of transporting liquid from the reservoir to the atmosphere and permitting its dissemination into the atmosphere. There is available a wide variety of such members. One popular example is a porous wick of the type widely used in air fresheners and the like. These are generally cylindrical and may be made of various fibrous and non-fibrous materials, for example cellulosic materials, porous plastics and sintered powdered materials such as metals or ceramics. Another example is a flat porous member moulded or stamped from a flat sheet of a suitable material, such as plastics or cardboard. A third embodiment is a member bearing on its surface capillary channels. Such an embodiment is described, for example, in United States Patent 4,913,350.

In wick-based apparatus known to the art, the wick is held stationary in one position with the lower end in the liquid and the upper end in the atmosphere, the wick passing from the reservoir to the atmosphere via an opening in the reservoir, often a neck formed therein. In this case, the liquid transfer member, be it wick or otherwise, is slidable within a sleeve, which passes through an opening in the reservoir. In one particular embodiment, the sleeve forms part of a plug, which is a liquid-tight fit within the opening. The sleeve is impermeable to the liquid, and, in another embodiment, is made of the same material as the reservoir. This may be a plastics material.

At its lower end, the transfer member is impermeable to the liquid, that is, if the only part of the transfer member that is exposed to the liquid is this lower part, no liquid would be transferred. This can be achieved in any suitable manner, and a wide variety of methods are available, depending on the nature of the transfer member. The skilled person will readily be able to realise these, but some non-limiting examples include impregnating the lower end of the transfer member with an impermeable substance, fitting a cap of an impermeable material thereon and compressing the lower portion so that it is no longer permeable. A cap may be fitted by any convenient means. It may, for example, be fitted directly to the transfer member, if the transfer member is sufficiently rigid and/or strong. Alternatively, it may be attached to a rigid axial rod within the transfer member, or to a rigid, open structure external to the transfer member. In the case of external capillary transfer members, such as those described in US 4,913,350, the liquid-impermeable lower end may be formed integrally with the member, in a single operation.

This liquid-impermeable lower end is adapted to cooperate with the sleeve, such that, when the transfer member is in an upper position in the sleeve, access of the liquid to the transfer member is denied. This confers a hitherto unattainable flexibility on such apparatus. For example, the apparatus is leakproof when not in use, and can be safely transported. It also means that, unlike conventional apparatus, it can be prevented from emitting liquid when emission is not desired. This can be arranged by any suitable means, and the skilled person will be able to envisage many possibilities. For example, if the transfer member is terminated at its lower end by an impermeable cap, this cap may be dimensioned so that it fits like a plug into an end of the sleeve. In one particular embodiment, the sleeve and the cap are both of cylindrical cross-section.

The reservoir comprises at the bottom thereof a cavity dimensioned to accommodate at least the impermeable part of the transfer member. This allows substantially all of the liquid to reach the transfer member and pass to the atmosphere. Such a feature can easily be provided, for example, by stamping, boring or moulding.

An important feature of the invention is that the transfer member be held in the raised position by suitable releasable locking means, such that, when released, it allows the transfer member to be moved with respect to the sleeve to a lowered position in liquid transfer contact with the liquid. Again, the skilled person will be able to envisage a wide range of ways of achieving such a result. For example, the sleeve and the end cap mentioned in the previous paragraph may be dimensioned such that one is a tight friction fit within the other, and a suitably energetic pulling or pushing will respectively lock or unlock the transfer member.

However, in one particular embodiment, the locking means is a more positive means. There are many possible means involving, for example, spring-loaded releasable catches, and such means work well, but for reasons of simplicity and cost, one particular embodiment uses materials that are naturally resilient and that can be moulded in the appropriate shapes. For example, one such means is to provide on the transfer means a protrusion that engages with a depression in a member placed adjacent to the transfer means, one or both of protrusion and depression-bearing member being sufficiently resilient such that a suitable pressure will engage or disengage them. It is of course possible to do the reverse, provide the protrusion on the member and the depression on the transfer member.

In a further particular embodiment of the mechanism described in the previous paragraph, the protrusion and depression are equipped with profiles that allow an easier movement in one of the two possible directions. This may be achieved, for example, by giving the protrusion an asymmetric cross-section, such that it has a "nose" profile, for example, with one side of the protrusion making a larger angle at the axis of the wick than the other, and the depression having a similar profile. In such a case, the smaller angle side will slide into the sleeve more easily than will the larger angle side, and a preferred orientation can be selected. For example, it may be desired for security reasons that a transfer member is more easily slipped into a sleeve than out again.

In one embodiment, the protrusion is a circumferential ridge and the depression is a matching circumferential groove, one being on the transfer member, the other on a member that circumscribes the transfer member.

In a particular embodiment, the circumferential ridge hereinabove described forms part of an impermeable cap at the lower end of the transfer means and this interacts with a corresponding groove formed in the sleeve within which the transfer means slides. Alternatively, the ridge may be in the transfer means and the groove in the cap. This serves not only to lock the transfer means in place in the upper position, but also to assist in the forming of a liquid-tight seal, such that the apparatus is leakproof.

The apparatus as hereinabove described can be used in this form as a dispenser of volatile liquids, the apparatus being supplied with the transfer member in the raised position, so that no liquid can escape. Such an apparatus will usually be equipped with a cap, which protects that part of the transfer member that will be exposed to the atmosphere and keeps it clean. In one particular embodiment, the interior of the cap is provided with means that interlock with corresponding means on the top of the transfer member. This has the effect of holding the transfer member securely in the raised position. The means can be, for example, co-operating screw threads, or a ridge/groove mechanism as hereinabove described with reference to the holding together of the liquid-impermeable lower end and the sleeve, or any other convenient mechanism.

The apparatus can also be used as a refill for a liquid dispensing unit into which it can fit. The dispensing unit can take many forms, and can include other elements, for example fans or heaters to assist in the dissemination of the liquid into the atmosphere. In a particular embodiment, refill and dispensing unit comprise means that compel the transfer member of a refill properly fitted to the dispensing unit to be in the lowered position, such that liquid can escape via the transfer member. This can be achieved by any suitable means. For example, the refill may be attached to the dispensing unit by a refill holding means, for example, screw threads and snap fitting, the dispensing unit additionally providing an opening element that acts on the transfer member to push it into a lowered position. The opening element may be a simple, downwardly-extending protrusion, or it may comprise means capable of locking with the upper part of the transfer member, such that, when the refill is removed, the transfer member is pulled upwards until the liquid-impermeable end and the sleeve come into locking correspondence, such that there can be no leakage. This opening element may comprise features that permit only the use of desired refills, for example, a protrusion that fits into a matching recess in the transfer member, such that refills lacking this recess can not be fitted.

The apparatus of this invention are easy and inexpensive to manufacture and use, and they are effective in use. The closed apparatus is leakproof, and it allows liquid to be disseminated into an atmosphere when required, advantages not found in conventional apparatus using transfer means such as porous wicks.

An additional problem that may be solved by this invention is that of "habituation", that is, the tendency of people to get used to a particular fragrance and therefore no longer notice it. This can be minimised by changing the fragrance frequently, and one way of achieving this is to have systems that diffuse more than one fragrance. The change between fragrances can be manual or automatic. For example, in a simple manual system, one refill as hereinabove described may be easily exchanged for another containing a different fragrance. The invention ensures the refill taken out does not leak whilst not in use, and it can be put back in when a fragrance change is desired. Alternatively, a fragrancing system may contain a plurality of such refills, which may be opened and closed automatically, according to a predetermined program. The advantages of non-leaking are again realised.

The invention is further described with reference to the accompanying drawing, which depicts a preferred embodiment and which is not to be considered limiting in any way.
Figure 1 depicts a partial vertical cross-section of an apparatus according to the invention.
Figure 2 depicts a cross-section of a detail of a particular embodiment.

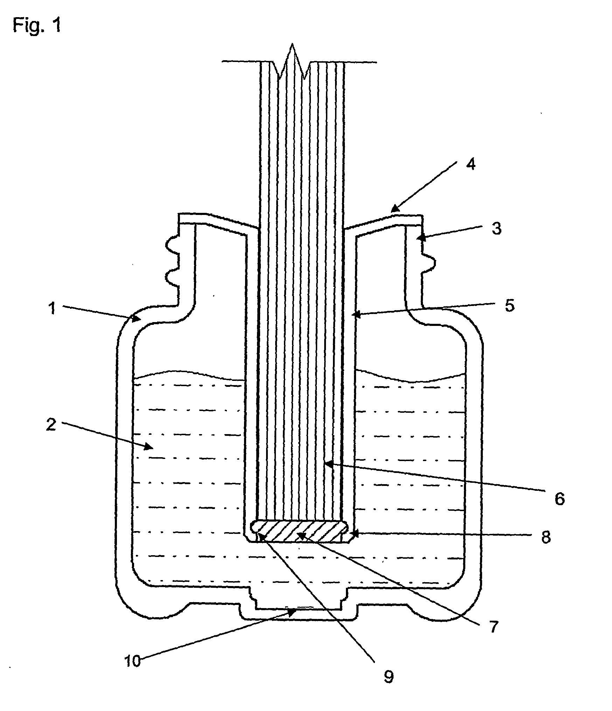

In Figure 1, a reservoir 1 contains a volatile liquid 2. The reservoir has a neck 3, within which is fitted an insert 4, which serves to block the neck and prevent undesired escape of liquid. Formed as part of this insert is a cylindrical sleeve 5 of circular cross-section, which is coaxial with the neck and which extends downwards from the neck into the liquid. Slidably mounted within the sleeve is a wick 6, which is capable of transferring liquid from the reservoir to the atmosphere. The wick as depicted here is a surface capillary type, as described in United States Patent 4, 913,350, but a conventional porous wick, of the type commonly used in commercially-available air fresheners, can easily be substituted. On the end of this wick is mounted a continuation 7, made of liquid-impermeable material and having generally the same radius as the wick. On its surface, the continuation bears a circumferential ridge 8, which is adapted to fit into a circumferential groove 9 formed in the lower end of the internal wall of the sleeve 5. When the ridge 8 is positioned within the groove 9, the wick 6 is held in a fixed position with respect to the sleeve. Either or both of the sleeve and the continuation are made of a resilient polymeric material, such that the ridge and the groove may be snapped out of contact. The bottom of the reservoir comprises a well 10, which is dimensioned to receive the continuation 7. Thus, substantially all of the liquid can reach the transfer member and be released into the atmosphere.

In operation, when the ridge 8 fits within the groove 9, sleeve 5 and continuation 8 present to the liquid an impermeable barrier, such that liquid cannot access the transfer member and escape to the atmosphere. Downwards pressure on the wick 6 causes the ridge 8 to snap out of the groove 9, thus allowing the wick 6 to be moved downwards into liquid transfer contact with the liquid. When it is desired to stop liquid transfer contact, pulling the wick upwards will cause the ridge 8 to snap into the groove 9, thus again presenting to the liquid an impermeable barrier.

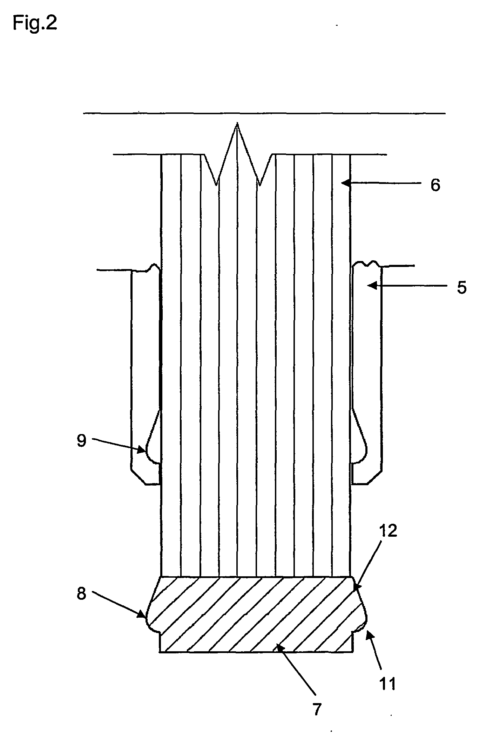

Figure 2 shows a detail of a particular ridge and groove construction. In this case, the ridge 8 has an asymmetric "inverted nose" profile, with the lower part 11 making a larger angle with the axis of the wick than does the upper part 12. The profile of the groove 9 matches that of the ridge 8. This means that the wick and sleeve combination is more easily closed than opened, an inherently more secure arrangement.

## Claims

1. An apparatus adapted to disseminate volatile liquid into an atmosphere, the apparatus comprising a reservoir (1) containing volatile liquid (2) and a liquid transfer member (6) adapted to have contact with both liquid and atmosphere, whereby the transfer member is sliding within a liquid-impermeable sleeve (5) and has a liquid-impermeable lower end (7), the liquid-impermeable lower end and liquid-impermeable sleeve combining, when the transfer member is in a raised position, to deny access of the liquid to the transfer member, the transfer member being held in the raised position by suitable releasable locking means (8,9) that, when released, allows the transfer member to be moved with respect to the sleeve to a lowered position in liquid transfer contact with the liquid, **characterised in that** the reservoir comprises at the bottom thereof a cavity dimensioned to accommodate at least the impermeable part of the transfer member.

2. An apparatus according to claim 1, in which the lower impermeable end (7) comprises a construction selected from the group consisting (a) an end impregnated with an impermeable substance (b) a cap of an impermeable material, and (c) a lower portion compressed to the point of impermeability.

3. An apparatus according to claim 2, in which the lower impermeable end comprises a cap adapted to fit like a plug into the end of the sleeve.

4. An apparatus according to claim 1, in which the locking means comprises on one of the transfer means and a member adj acent to the transfer means a protrusion that engages with a depression in the other of the transfer means and adjacent member, one or both of protrusion and depression-bearing member being sufficiently resilient such that a suitable pressure will engage or disengage them.

5. An apparatus according to claim 4, in which the protrusion is a circumferential ridge (8) and the depression is a matching circumferential depression (9).

6. An apparatus according to claim 5, in which the circumferential ridge (8) forms part of one of an impermeable cap at the lower end of the transfer means and a sleeve in which the transfer means slides, and the corresponding circumferential depression (9) is formed in the other one of cap and sleeve.

7. An apparatus according to claim 4, in which protrusion and depression have matching asymmetric profiles permitting easier movement in one sliding direction.

8. A refillable volatile liquid dispensing apparatus comprising as exchangeable refill at least one apparatus according to claim 1, the refillable apparatus comprising means that open the refill as it is inserted, by causing the transfer member to be lowered into liquid transfer contact with the liquid.

9. A refillable volatile liquid dispensing apparatus according to claim 8, in which the refillable apparatus comprises means that cause the refill to close as it is removed from the refillable apparatus, by causing the transfer member to move into a raised position such that its impermeable end combines with the impermeable sleeve to deny access of the liquid to the transfer member.

10. A method of reversibly preventing the escape from a reservoir (1) of volatile liquid (2), which is contained in an apparatus according to claims 1-9, that is adapted to be disseminated into an atmosphere from the reservoir by means of a liquid transfer member (6) that extends from the liquid in the reservoir to the atmosphere; **characteristed in that** it comprises
(a) rendering liquid-impermeable part of that end of the transfer member that may contact the liquid;
(b) rendering the transfer member slideable in a liquid-impermeable sleeve (5), such that the transfer member has a raised and a lowered position, the lowered position permitting liquid transfer contact of the transfer member with the liquid;
(c) configuring the liquid-impermeable part of the transfer member and the impermeable sleeve, such that, in the raised position, they are releasably locked together to form a liquid-impermeable barrier denying access of the liquid to the transfer means; and
(d) sliding the transfer member to the raised position.

## Patentansprüche

1. Vorrichtung zur Verteilung einer flüchtigen Flüssigkeit in eine Atmosphäre, wobei die Vorrichtung einen Behälter (1), der eine flüchtige Flüssigkeit (2) enthält, und ein Flüssigkeitsübertragungsglied (6) umfasst, das dazu ausgeführt ist, Kontakt sowohl mit der Flüssigkeit als auch mit der Atmosphäre zu haben, wobei das Übertragungsglied in einer flüssigkeitsundurchlässigen Hülse (5) verschiebbar ist und ein flüssigkeitsundurchlässiges unteres Ende (7) aufweist, wobei das flüssigkeitsundurchlässige untere Ende und die flüssigkeitsundurchlässige Hülse zusammenwirken, wenn das Übertragungsglied in einer angehobenen Position ist, um der Flüssigkeit Zugang zum Übertragungsglied zu verwehren, wobei das Übertragungsglied durch ein geeignetes, freigebbares Verriegelungsmittel (8, 9) in der angehobenen Position gehalten wird, wobei das Verriegelungsmittel, wenn es freigegeben ist, eine Bewegung des Übertragungsglieds bezüglich der Hülse in eine abgesenkte Position in Flüssigkeitsübertragungskontakt mit der Flüssigkeit gestattet, **dadurch gekennzeichnet, dass** der Behälter an seinem Boden einen Hohlraum umfasst, der zur Aufnahme mindestens des undurchlässigen Teils des Übertragungsglieds dimensioniert ist.

2. Vorrichtung nach Anspruch 1, wobei das untere undurchlässige Ende (7) eine Konstruktion umfasst, die aus der aus (a) einem mit einer undurchlässigen Substanz getränkten Ende, (b) einer Kappe aus einem undurchlässigen Material und (c) einem bis zur Undurchlässigkeit komprimierten unteren Teil bestehenden Gruppe ausgewählt ist.

3. Vorrichtung nach Anspruch 2, wobei das untere undurchlässige Ende eine Kappe umfasst, die dazu ausgeführt ist, wie ein Stopfen in das Ende der Hülse zu passen.

4. Vorrichtung nach Anspruch 1, wobei das Verriegelungsmittel an dem Übertragungsmittel oder an einem Glied neben dem Übertragungsmittel einen Vorsprung umfasst, der mit einer Vertiefung in dem jeweils anderen Element, dem Übertragungsmittel oder dem benachbarten Glied, in Eingriff gelangt, wobei das den Vorsprung und/oder das die Vertiefung aufweisende Glied so elastisch sind/ist, dass ein geeigneter Druck sie in oder außer Eingriff bringt.

5. Vorrichtung nach Anspruch 4, wobei der Vorsprung ein Umfangssteg (8) und die Vertiefung eine damit zusammenpassende Umfangsvertiefung (9) ist.

6. Vorrichtung nach Anspruch 5, wobei der Umfangssteg (8) Teil einer undurchlässigen Kappe am unteren Ende des Übertragungsmittels oder einer Hülse bildet, in der das Übertragungsmittel gleitet, und die entsprechende Umfangsvertiefung (9) in dem jeweils anderen Element, in der Kappe oder der Hülse, ausgebildet ist.

7. Vorrichtung nach Anspruch 4, wobei der Vorsprung und die Vertiefung zusammenpassende, asymmetrische Profile aufweisen, die eine leichtere Bewegung in einer Schieberichtung gestatten.

8. Nachfüllbare Vorrichtung zur Verteilung einer flüchtigen Flüssigkeit, die als austauschbare Nachfüllung mindestens eine Vorrichtung nach Anspruch 1 umfasst, wobei die nachfüllbare Vorrichtung Mittel umfasst, die die Nachfüllung bei ihrem Einsetzen öffnen, indem sie bewirken, dass das Übertragungsglied in Flüssigkeitsübertragungskontakt mit der Flüssigkeit abgesenkt wird.

9. Nachfüllbare Vorrichtung zur Verteilung einer flüchtigen Flüssigkeit nach Anspruch 8, wobei die nachfüllbare Vorrichtung Mittel umfasst, die bewirken, dass sich die Nachfüllung schließt, wenn sie aus der nachfüllbaren Vorrichtung entfernt wird, indem sie bewirken, dass sich das Übertragungsglied in eine angehobene Position bewegt, so dass sein undurchlässiges Ende mit der undurchlässigen Hülse zusammenwirkt, um der Flüssigkeit Zugang zum Übertragungsglied zu verwehren.

10. Verfahren zum reversiblen Verhindern des Entweichens einer flüchtigen Flüssigkeit (2), die in einer Vorrichtung nach den Ansprüchen 1 - 9 enthalten ist, aus einem Behälter (1), wobei die Flüssigkeit durch ein Flüssigkeitsübertragungsglied (6), das sich von der Flüssigkeit in dem Behälter zur Atmosphäre erstreckt, aus dem Behälter in eine Atmosphäre verteilt wird, **dadurch gekennzeichnet, dass** es Folgendes umfasst:
(a) Flüssigkeitsundurchlässigmachen eines Teils des Endes des Übertragungsglieds, das die Flüssigkeit berühren kann;
(b) Verschiebbarmachen des Übertragungsglieds in einer flüssigkeitsundurchlässigen Hülse (5) derart, dass das Übertragungsglied eine angehobene und eine abgesenkte Position aufweist, wobei die abgesenkte Position einen Flüssigkeitsübertragungskontakt des Übertragungsglieds mit der Flüssigkeit gestattet;
(c) Konfigurieren des flüssigkeitsundurchlässigen Teils des Übertragungsglieds und der undurchlässigen Hülse derart, dass sie in der angehobenen Position lösbar miteinander verriegelt sind, um eine flüssigkeitsundurchlässige Sperre zu bilden, die der Flüssigkeit Zugang zu dem Übertragungsmittel verwehrt; und
(d) Verschieben des Übertragungsglieds in die angehobene Position.

## Revendications

1. Appareil prévu pour diffuser un liquide volatil dans l'atmosphère, l'appareil comprenant un réservoir (1) contenant le liquide volatil (2) et un organe de transfert de liquide (6) prévu pour être en contact à la fois avec le liquide et avec l'atmosphère, l'organe de transfert glissant dans un manchon imperméable aux liquides (5) et ayant une extrémité inférieure imperméable aux liquides (7), l'extrémité inférieure imperméable aux liquides et le manchon imperméable aux liquides coopérant, lorsque l'organe de transfert est dans une position relevée, pour empêcher l'accès du liquide à l'organe de transfert, l'organe de transfert étant maintenu dans la position relevée par des moyens de verrouillage libérables appropriés (8, 9) qui, lorsqu'ils sont libérés, permettent à l'organe de transfert d'être déplacé par rapport au manchon dans une position abaissée en contact de transfert liquide avec le liquide, **caractérisé en ce que** le réservoir comprend dans son fond une cavité dimensionnée pour recevoir au moins la partie imperméable de l'organe de transfert.

2. Appareil selon la revendication 1, dans lequel l'extrémité imperméable inférieure (7) comprend une construction choisie parmi le groupe constitué (a) d'une extrémité imprégnée d'une substance imperméable, (b) d'un capuchon d'un matériau imperméable, et (c) d'une portion inférieure comprimée au point de devenir imperméable.

3. Appareil selon la revendication 2, dans lequel l'extrémité imperméable inférieure comprend un capuchon prévu pour s'ajuster comme un bouchon dans l'extrémité du manchon.

4. Appareil selon la revendication 1, dans lequel le moyens de verrouillage comprend, sur un élément parmi le moyen de transfert et un organe adjacent au moyen de transfert, une saillie qui s'engage avec une dépression dans l'autre élément parmi le moyen de transfert et l'organe adjacent, la saillie et/ou l'organe portant la dépression étant suffisamment élastiques pour qu'une pression appropriée puisse les engager l'un dans l'autre ou les désengager l'un de l'autre.

5. Appareil selon la revendication 4, dans lequel la saillie est une arête circonférentielle (8) et la dépression est une dépression circonférentielle correspondante (9).

6. Appareil selon la revendication 5, dans lequel l'arête circonférentielle (8) fait partie d'un capuchon imperméable à l'extrémité inférieure du moyen de transfert ou d'un manchon dans lequel glisse le moyen de transfert, et la dépression circonférentielle correspondante (9) est formée dans l'autre élément, c'est-à-dire le capuchon ou le manchon.

7. Appareil selon la revendication 4, dans lequel la saillie et la dépression ont des profils asymétriques correspondants permettant un mouvement plus facile dans une direction de glissement.

8. Appareil rechargeable prévu pour diffuser un liquide volatil, comprenant en tant que cartouche de remplacement, au moins un appareil selon la revendication 1, l'appareil rechargeable comprenant des moyens qui ouvrent la recharge au moment de son insertion, en provoquant l'abaissement de l'organe de transfert en contact de transfert liquide avec le liquide.

9. Appareil rechargeable prévu pour diffuser un liquide volatil selon la revendication 8, dans lequel l'appareil rechargeable comprend des moyens qui causent la fermeture de la recharge à mesure qu'elle est retirée de l'appareil rechargeable, en provoquant le déplacement de l'organe de transfert dans une position relevée de telle sorte que son extrémité imperméable soit combinée avec le manchon imperméable pour empêcher l'accès du liquide à l'organe de transfert.

10. Procédé pour empêcher, de manière réversible, que du liquide volatil (2), qui est contenu dans un appareil selon les revendications 1 à 9, ne s'échappe d'un réservoir (1), ce liquide volatil étant prévu pour être diffusé dans une atmosphère à partir du réservoir au moyen d'un organe de transfert de liquide (6) qui s'étend depuis le liquide dans le réservoir jusqu'à l'atmosphère ; **caractérisé en ce qu'**il comprend les étapes suivantes :
(a) rendre imperméable aux liquides une partie de l'extrémité de l'organe de transfert qui peut venir en contact avec le liquide ;
(b) rendre l'organe de transfert capable de glisser dans un manchon (5) imperméable aux liquides, de telle sorte que l'organe de transfert ait une position relevée et une position abaissée, la position abaissée permettant à l'organe de transfert d'être en contact de transfert liquide avec le liquide ;
(c) configurer la partie imperméable aux liquides de l'organe de transfert et le manchon imperméable de telle sorte que dans la position relevée, ils soient verrouillés de manière amovible l'un à l'autre pour former une barrière imperméable aux liquides empêchant l'accès du liquide au moyen de transfert ; et
(d) faire glisser l'organe de transfert dans la position relevée.
